# EUROPEAN PATENT APPLICATION

(11) **EP 3 841 872 A1**
(43) Date of publication of application: **30.06.2021**
(21) Application number: 19383210.2
(22) Date of filing: 28.12.2019
(51) Int. Cl.: A01H 4/00, A01H 6/28

(54) **IN VITRO DIRECT REGENERATION OF POLYPLOID CANNABIS PLANTS**

(71) Applicant: Ploidy and Genomics SL., 46020 Valencia (ES)
(72) Inventor: Galán Ávila, Alberto, 46980 Paterna (Valencia) (ES)
(74) Representative: Carlos Hernando, Borja

(57) **Abstract**

The present invention relates to a new *in vitro* method for direct regeneration of a *Cannabis sativa* L. plant as well as the use of such method for micropropagation of selected elite clones belonging to *Cannabis sativa* L., development of polyploid *Cannabis* plants with enhanced levels of secondary metabolites, promote spontaneous rooting of *in vitro* regenerants in a shorter period of time than conventionally used methods, production of mutagenized plants and obtention of transgenic or gene-edited plants. The method advantageously comprises culturing selected explants such as apical meristems, true leaves, stem nodes and/or internodes from mature plants and cotyledons, hypocotyls and/or epicotyls from seedlings of short and neutral-day *Cannabis* plants in an appropriate culture medium.

## Description

### TECHNICAL FIELD OF THE INVENTION

The present invention is comprised in the agriculture, cosmetic and/or pharmaceutical industry and specifically relates to a new *in vitro* method for direct regeneration of a *Cannabis sativa* L. plant as well as the use of such method for micropropagation of selected elite clones belonging to *Cannabis sativa* L., development of polyploid *Cannabis* plants with enhanced levels of secondary metabolites, promote spontaneous rooting of *in vitro* regenerants in a shorter period of time than conventionally used methods, production of mutagenized plants and obtention of transgenic or gene-edited plants.

### BACKGROUND OF THE INVENTION

*Cannabis sativa* L. (2n=2x=20) is a dicotyledonous species belonging to Cannabaceae family used for multiple purposes (fiber, oil, edible seeds, medicinal drug) which comprises short and neutral-day varieties. Among its different applications, its use in medicine, derived from its content in cannabinoids (Cascio *et al.*, 2017) is recalling an increasing interest. Among cannabinoids, Δ9- tetrahydrocannabinol (THC) and cannabidiol (CBD) are generally the most abundant in the plant (Andre *et al.*, 2016). Recent research has reported many cannabinoid pharmacodynamic and pharmacokinetic properties, expanding the potential use of cannabinoids in medical therapies (Urits *et al.*, 2019) and promoting the development of *Cannabis* improved varieties with specific biochemical profiles.
In this respect, *in vitro* culture for producing *Cannabis* plants is a useful tool that has been employed to complement conventional breeding through large-scale micropropagation of selected elite clones (Lata *et al.*, 2017), development of polyploid varieties with enhanced levels of secondary metabolites (Mansouri and Bagheri, 2017; Parsons *et al.*, 2019) or genetic transformation of non-regenerating tissues (Feeney and Punja, 2003, 2015, 2017; Wahby *et al.*, 2017). However, there is still a lack of an *in vitro* regeneration protocol efficient in the broad range of genetically diverse materials in the species.
While there are hundreds of different active metabolites present in *Cannabis,* two cannabinoids are present in high concentrations, and are generally considered to be the most important: THC and CBD. THC is responsible for the well-known psychoactive properties of *Cannabis* whereas non-intoxicating CBD is widely used for pain, anxiety, depression, and sleep disorders. Another group of important chemicals is the terpenes, which contribute to the smell and taste of *Cannabis* products, but also function as active metabolites with therapeutic properties. All of these metabolites are produced and stored within glandular trichomes that mainly develop on the inflorescence of the plant. *Cannabis sativa* L. is a diploid species. Historically, new *Cannabis* strains have been developed through conventional breeding methods.
However, these methods can be imprecise, and require several generations before the desired traits are obtained and a stable strain is produced. One strategy to accelerate breeding development is a chromosome doubling event called polyploidization.
Polyploidization consists in an increase in the number of complete sets of chromosomes and is common in the plant kingdom, being associated with increased genetic diversity in some plant lineages. Desirable consequences of polyploidy for plant breeding include the buffering of deleterious mutations, increased heterozygosity, and hybrid vigor. Consequently, polyploids often have phenotypic traits that are distinct from diploids, including larger flowers or leaves. Increases in active metabolite concentration in polyploids are reported for numerous medicinal plants (lannicelli *et a*/*.*, 2019).
Polyploidy can be induced through *in vivo* or *in vitro* application of antimitotic agents to seeds, seedlings or shoot tips.
*Cannabis* strains are recalcitrant to *in vitro* shoot regeneration protocols. *Cannabis* strains are not genetically stable when propagated through seeds, and while there has been little success in regenerating *Cannabis* shoots from callus, (non direct *in vitro* regeneration process), the propagation of high THC drug-type *Cannabis* through tissue culture using nodal explants has been described to be not economically feasible.
In this respect, plant regeneration is an essential step for most *in vitro* culture techniques employed in plant breeding. Although high rates of *in vitro* plant regeneration from apical and axillary meristems of the plant (Richez-Dumanois *et al.*, 1986; Lata *et al.*, 2009, 2016), young leaves (Lata *et al.*, 2010) and cotyledons (Chaohua *et al.*, 2016) have already been reported, several studies point out to a high level of recalcitrance of *in vitro* shoot regeneration from different tissues, such as maturing bracts, anther-calyx complexes and vegetative leaves (Hemphill *et al.*, 1978), leaves, hypocotyls, cotyledons and roots (Mandolino and Ranalli, 1999), young leaves, petioles, internodes and axillary buds (Lusarkiewicz-Jarzina *et al.*, 2005), roots, leaves and stems (Plawuszewski *et al.*, 2006), cotyledons, stems and roots (Wielgus *et al.*, 2008), cotyledons and epicotyls (Movahedi *et al.*, 2015), leaves and hypocotyls (Movahedi *et al.*, 2016a, 2016b), and hairy roots (Wahby *et al.*, 2017).

Therefore, the low regeneration efficiency of published (non-direct) *in vitro* plant regeneration protocols for *Cannabis sativa* L. present a major drawback for the application of *in vitro* tissue culture to the improvement of *C. sativa.* Moreover, in most of the aforementioned publications, when shoot regeneration was successful, it developed in an indirect way through a previous phase of callus formation, which compromises the genetic fidelity of regenerants with respect to the donor plant. In addition, a common feature that can be inferred from the studies previously mentioned is that the development of *in vitro* shoot organogenesis in this species follows only with the required addition of plant growth regulators to the culture medium.
US20190230882 refers to methods for producing a *Cannabis* plant by crossing a first parent *Cannabis* plant with a second parent *Cannabis* plant, wherein the first parent *Cannabis* plant or second parent *Cannabis* plant is the *Cannabis* plant from cultivar NWG331 or NWG452, wherein a representative sample of seed of said cultivar was deposited under Accession No. NCIMB 43290 or NCIMB 43280.
WO2019006466 refers to a set of media for producing a hemp plant or plant part wherein the set of media comprises: (a) one or more initiation medium; (b) one or more multiplication medium; and (c) one or more rooting medium; wherein the initiation medium, the multiplication medium and/or the rooting medium comprises at least one cytokinin; wherein the media is selected from one of BOO 1 - B0091. US20190289804 discloses a method for inducing polyploidy in a *Cannabis* plant, the method comprising treating the *Cannabis* plant or a part thereof with an amount of a dinitroaniline compound effective to induce polyploidy.
WO2019006470 discloses a medium for producing *cannabis* micropropagation wherein said medium comprises sucrose and (a) at least one cytokinin and at least one auxin; (b) at least one growth retardant; or (c) at least one cytokinin, at least one auxin, and at least one growth retardant.
US20180258439 refers to a method for altering a content of one or more cannabinoids in a *cannabis* plant, the method comprising down-regulating activity of tetrahydrocannabinolic acid (THCA) synthase in the *Cannabis* plant.

The present invention provides a new method for *in vitro* direct regeneration of polyploid *Cannabis* plants which overcomes the drawbacks known from the prior art methods and present unexpected advantages in terms of quality, efficiency and time required for *in vitro* production of plants. The method of the present invention ensures the genetic fidelity of regenerants with respect to the donor plant, increased ploidy levels of regenerants and provides a method for obtaining spontaneous rooting of regenerants in a shorter period of time than conventionally used methods, and for producing mutagenized plants and transgenic or gene-edited plants.

### BRIEF DESCRIPTION OF THE INVENTION

Development of improved varieties with specific biochemical profiles is one of the main goals in *Cannabis sativa* L. breeding. *In vitro* shoot regeneration can efficiently contribute to the improvement of *C. sativa* through micropropagation, development of polyploids, obtention of mutagenized plants or production of transgenic or gene-edited plants. However, *C. sativa* is considered as recalcitrant to *in vitro* shoot regeneration.
The author of the present invention has developed a highly efficient protocol or method for *in vitro* direct regeneration of polyploid *Cannabis sativa* L. plants from apical meristems, stem nodes, internodes, true leaves, cotyledons, hypocotyls and/or epicotyls used as explants obtained from donor *Cannabis* plant seedlings or mature *Cannabis* plants of short and neutral-day varieties such as Ferimon, Felina32, Fedora 17, USO31 and Finola.

Based on the experimental results contained in the present specification, one embodiment of the present invention is directed to a method for *in vitro* direct regeneration of polyploid plants coming from *Cannabis sativa* L. by means of *in vitro* tissue culture of apical meristems, stem nodes, internodes or true leaves from a mature *Cannabis* plant or cotyledons, hypocotyls or epicotyls from a *Cannabis* seedling, said method characterized by:
- comprising *in vitro* culturing of apical meristem, stem node, internode, true leaf, cotyledon, hypocotyl and/or epicotyl explants of a donor *Cannabis* plant under conditions for promoting the growing of embryos, shoots and/or shoots with roots; and
- preventing the formation of an intermediate callus phase.

A further embodiment of the present invention is a method for *in vitro* direct regeneration of a *Cannabis* plant as in preceding paragraph, characterized in that the apical meristem, stem node, internode, true leaf, cotyledon, hypocotyl or epicotyl explants are dissected from a *Cannabis* donor plant with a phenological growth stage coded from 00 to 99 according to BBCH-scale of Mishchenko *et a*/*.*, (2017).In a preferred embodiment, the explants are dissected from seven-days-old seedlings of a *Cannabis sativa* L. donor plant.

A further embodiment of the present invention is directed to a method as defined in any of the preceding paragraphs which is characterized in that the culture medium consists of macronutrients, micronutrients, vitamins and carbon sources, with or without gelling agents, plant growth regulators and chemical microtubule disruptors.

A further embodiment of the present invention is directed to a method as defined in any of the preceding paragraphs which is characterized in that the culture medium for *in vitro* culturing of apical meristem, true leaf, stem node, internode, hypocotyl and/or epicotyl explants is hormone-free.

A further embodiment of the present invention is directed to a method as defined in any of the preceding paragraphs which is characterized in that the culturing medium consists of macronutrients, micronutrients, vitamins, carbon sources and gelling agents.

A further embodiment of the present invention is directed to a method as defined in any of the preceding paragraphs which is characterized in that the explants are grown at 22°C ± 5°C and 60% ± 10% relative humidity.

A further embodiment of the present invention is directed to a method as defined in any of the preceding paragraphs which is characterized in that the photoperiod during culturing consists of between 16 and 18 hours of light and between 6 and 8 hours of dark.

A further embodiment of the present invention is directed to a method as defined in any of the preceding paragraphs which is characterized in that the explants are *in vitro* cultured during at least 2 weeks.

A further embodiment of the present invention is directed to a method as defined in any of the preceding paragraphs characterized in that it further comprises a step after culturing the explants where shoots and/or embryos regenerated from the original explant are subcultured individually to glass-tubes, vessels and/or containers of multiple volumes to produce rooted *Cannabis* plants.

A further embodiment of the present invention is directed to a method as defined in any of the preceding paragraphs characterized by further comprising a step prior to *in vitro* culturing of cotyledon, hypocotyl and/or epicotyl explants, where seeds of the donor *Cannabis* plant are surface sterilized and washed in autoclaved water.

A further embodiment of the present invention is directed to a method as defined in the preceding paragraph which is characterized in that once sterilized the seeds are germinated to produce a plant with a phenological growth stage coded from 00 to 99 according to BBCH-scale of Mishchenko *et al.* (2017). More preferably, the seeds are germinated to produce seven-days-old seedlings.

A further embodiment of the present invention is directed to a method as defined in any of the preceding paragraphs which is characterized in that it optionally comprises a step after culturing the explants where shoots and or embryos regenerated from the original explant are sub-cultured individually to glass-tubes, vessels or containers of multiple volumes to produce rooted *Cannabis* plants or for promotion of embryo conversion into seedlings. Such glass-tubes, vessels or containers may be of different volumes.

A further embodiment of the present invention is directed to a method as defined in any of the preceding paragraphs which is characterized by comprising a further step after sub-culturing the explants presenting shoots where rooted *Cannabis* plants or embryo-derived plantlets are transplanted in pots with fertilized substrate and are subjected to acclimatization. A further method of the present invention comprises directly transplanting rooted plants obtained after culturing the explants in pots with fertilized substrate and subsequent acclimatization.

A further embodiment of the present invention is directed to a method as defined in any of the preceding paragraphs which is characterized in that the donor *Cannabis* plant belongs to *Cannabis sativa* L.

A further embodiment of the present invention is directed to a *Cannabis* plant regenerant obtainable according to the method described in the present invention as defined in preceding paragraphs.

A further embodiment of the present invention is directed to a method for micropropagation of selected elite clones belonging to *Cannabis sativa* L. which comprises the method for *in vitro* direct regeneration of *Cannabis* plants of the present invention as defined in preceding paragraphs.

A further embodiment of the present invention is directed to a method for obtaining a polyploid *Cannabis* plant and/or modifying ploidy level of a *Cannabis* plant which comprises the method for *in vitro* direct regeneration of a *Cannabis* plant of the present invention as defined in preceding paragraphs.

A further embodiment of the present invention is directed to a method for obtaining transgenic or gene-edited *Cannabis* plants which comprises the method for *in vitro* direct regeneration of a *Cannabis* plant of the present invention as defined in preceding paragraphs.

A further embodiment of the present invention is directed to a method for obtaining mutagenized *Cannabis* plants which comprises the method for *in vitro* direct regeneration of a *Cannabis* plant of the present invention as defined in preceding paragraphs.

The method of the present invention provides the following unexpected advantages:
- provides spontaneous rooting in *Cannabis* regenerants,
- provides rooted *Cannabis* plantlets in about 2 - 4 weeks from *in vitro* culture of the original explant,
- ensures the genetic fidelity of the *Cannabis* regenerants/plants obtained with respect to the *Cannabis* donor plant,
- increases ploidy level in the *Cannabis* regenerants/plants obtained without addition of plant growth regulators and chemical microtubule disruptors to the culture medium. Mixoploid (with 2n and 4n cells) plants are obtained in around 20% of regenerated plants.

In a preferred embodiment, the present invention is therefore directed to a method for *in vitro* direct regeneration of *Cannabis* plants, which comprises the following steps:
a) Collecting seeds of a donor *Cannabis sativa* L. plant;
b) Surface sterilization of the seeds;
c) Germination of the seeds in a Petri dish containing germination medium;
d) Dissection of explants from a plant with a phenological growth stage coded from 00 to 99 according to BBCH-scale of Mishchenko *et al.* (2017), or preferably, from seven-days-old seedlings of a donor *Cannabis* plant;
e) Culturing of the explants in a culture medium under controled conditions of temperature and relative humidity during 2 - 3 weeks;
f) Selection of specimens with shoot and roots;
g) Sub-culturing the specimens without roots individually to glass-tubes, vessels or containers of different volumes with the culture medium used in step e) until spontaneously rooted plants are generated;
h) Transplanting spontaneously rooted plants in pots with fertilized substrate and acclimatizing as needed,
wherein the explants used in step e) are selected from apical meristems, true leaves, stem nodes, internodes, cotyledons, hypocotyls and/or epicotyls and wherein the method prevents the formation of an intermediate callus phase.

In a further preferred embodiment of the present invention, the method according to the present invention, as described in any preceding paragraph, comprises culturing the explants in a hormone-free culture medium.

For the purpose of the present invention the following terms have the following meaning:
The term "*Cannabis* plant" is used with reference to a genus of flowering plants in the family Cannabaceae, which contains at least 3 subspecies: *Cannabis sativa* L. ssp. sativa, *Cannabis sativa* L. ssp. indica, and *Cannabis sativa* L. ssp. ruderalis.

The term "donor *Cannabis* plant" refers to a selected young heathy plant exhibiting a desired phenotype, chemotype and/or genotype which is typically maintained in a vegetative stage throughout its entire life and has passed rigorous testing for diseases or a newly-germinated seedling from a specific *C. sativa* variety.

The term "mature plant" as used herein refers to *Cannabis sativa* L. plants in a phenological growth stage coded from 12 to 99 according to BBCH-scale of Mishchenko *et al.* (2017).

The term "explant" as used herein refers to plant tissue, means living plant tissue that is removed from the natural site of growth and placed in sterile medium (e.g., DKW or MS) for *in vitro* culture under aseptic conditions. This can be of any tissue type such as leaves, roots, stems, or any portion taken from a plant and used to initiate tissue culture. In particular, the present invention contemplates the use of apical meristems, stem nodes, internodes, hypocotyls, epicotyls, cotyledons and true leaves.

The term "apical meristem" as used herein, since it is located on the top of the stem and above cotyledons, is equivalent to the term "epicotyl" and refers to the cluster of meristematic cells capable of differentiate into distinct plant organs and/or multicellular structures such as embryos, shoots and/or leaves. It is located on the top of the main stem of the plant and on the top of secondary branches of the plant.

The term "stem node" as used herein, since it is located above cotyledons, is equivalent to the term "epicotyl" and refers to the portion of stem from which branches comprising stems, petioles, leaves, flowers and/or apical meristems are originated.

The term "internode" as used herein, since it is located above cotyledons, is equivalent to the term "epicotyl" and refers to the portion of the stem located between stem nodes and/or between stem nodes and apical meristems.

The term "hypocotyl" as used herein refers to the portion of the stem located under cotyledons and above radicule.

The term "epicotyl" as used herein refers to the portion of the stem located above cotyledons.

The term "cotyledon" as used herein refers to a pair of rounded leaves present in the seedling even when it is enclosed inside the seed, whose function is to provide energy to the seedling while it is not able to develop photosynthesis by itself.

The term "true leaf" as used herein refers to a vegetative organ of a vascular plant responsible for performing photosynthesis and its corresponding petiole by which the leaf is attached to the stem.

The term "culture medium" as used herein refers to the medium in which explants are cultured. Its composition includes, but is not limited to macronutrients, micronutrients, vitamins, carbon sources, antioxidants (optional), gelling agents (optional) and plant growth regulators (optional).

The term "hormone-free culture medium" as used herein refers to a culture medium free of any natural or synthetic hormone, plant growth regulator, growth factor and any other substance or composition which promotes formation or development of any multicellular structure or organ in a plant (e.g. embryo, shoot and/or root).

The term "direct *in vitro* regeneration method" as used herein refers to an *in vitro* method of generating embryos, shoots and/or rooted plants or plantlets which does not involve the formation of a callus phase.

The term "plant regenerant" or "regenerant" as used herein refers to plants or plantlets regenerated through *in vitro* embryogenesis and/or shoot organogenesis.

### DESCRIPTION OF THE FIGURES

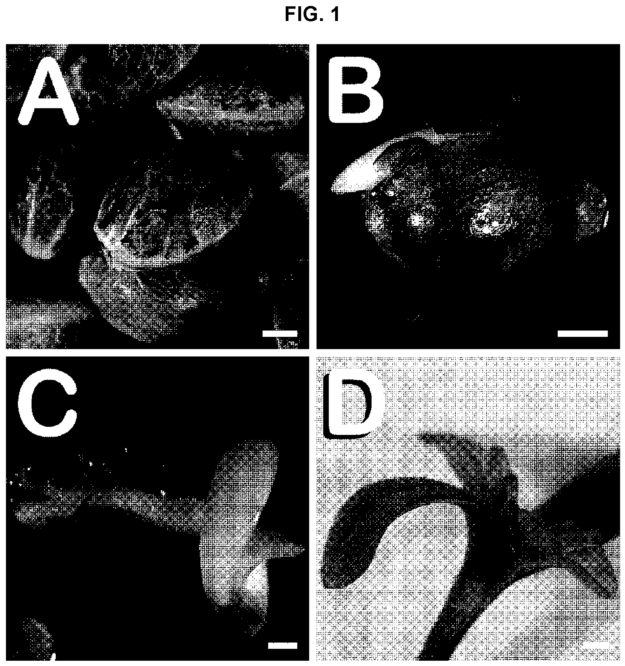

Figure 1: Seed germination of *C. sativa.* The different developmental stages are described as follows: Seeds just before being sterilized (A); germinated seed 48 h. after *in vitro* sowing with the root apical meristem arising from testa (B); emerging seedling 5 days after seed plating, with testa being visible at the bottom of the image (C); Seven-days-old seedling with fully expanded first pair of true leaves (D). Scale bars: 1 mm.

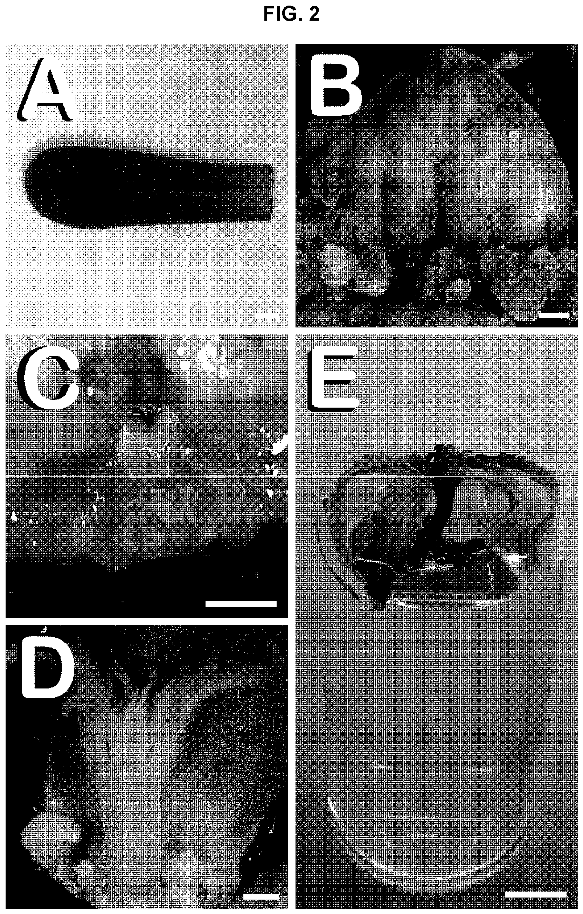

Figure 2: Direct *in vitro* shoot organogenesis from cotyledons of *C. sativa.* The different developmental stages are described as follows: Newly dissected cotyledon from a seven-days-old hemp seedling (A); Non-responding cotyledon with brown coloration after two weeks in culture where non-organogenic green callus proliferation is visible (B); Shoot primordium formation at the basal zone of cotyledon after four days of *in vitro* culture (C); Vigorous shoot arising from the lower part of cotyledon 14 days after exposure to the culture medium (D); Cotyledon derived plant cultured in a glass-tube 21 days after explant inoculation (E). Scale bars (A-D): 1 mm. Scale bar (E): 6 mm.

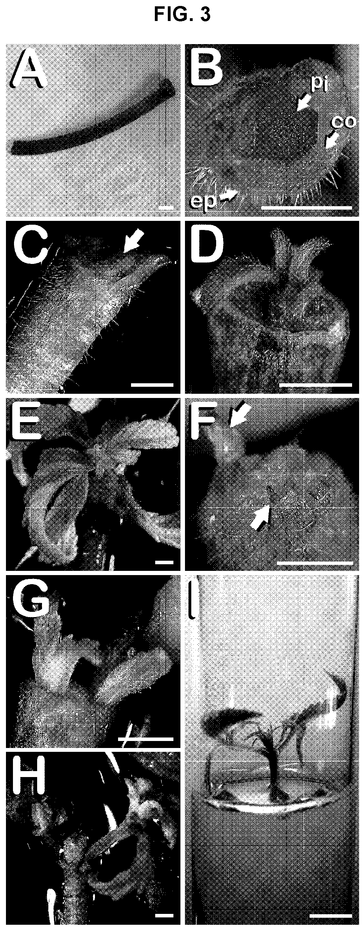

Figure 3: Direct *in vitro* shoot organogenesis from hypocotyls of *C. sativa.* The different developmental stages are described as follows: Newly dissected hypocotyl from a seven-days-old hemp seedling (A). Transverse section of newly dissected hemp hypocotyl revealing its different layers: ep: epidermis; co: cortex; pi: pith (B). Non-responding hypocotyl after two weeks in culture showing tissue oxidation on the top of the explant (arrow) (C). Formation of one shoot at the top of the hypocotyl after seven days of *in vitro* culture (D). Vigorous shoot arising from the upper part of hypocotyl 14 days after exposure to the culture medium (E). Two primordia arising from the top of the hypocotyl after four days of *in vitro* culture: Arrows point both primordia (F). Two hypocotyl derived plants nine days after explant inoculation (G). Two hypocotyl derived regenerants ready to be subcultured 14 days after explant culture (H). Hypocotyl derived plant individually grown in a glass-tube 21 days after culture initiation (I). Scale bars (A-H): 1 mm. Scale bar (I): 6 mm.

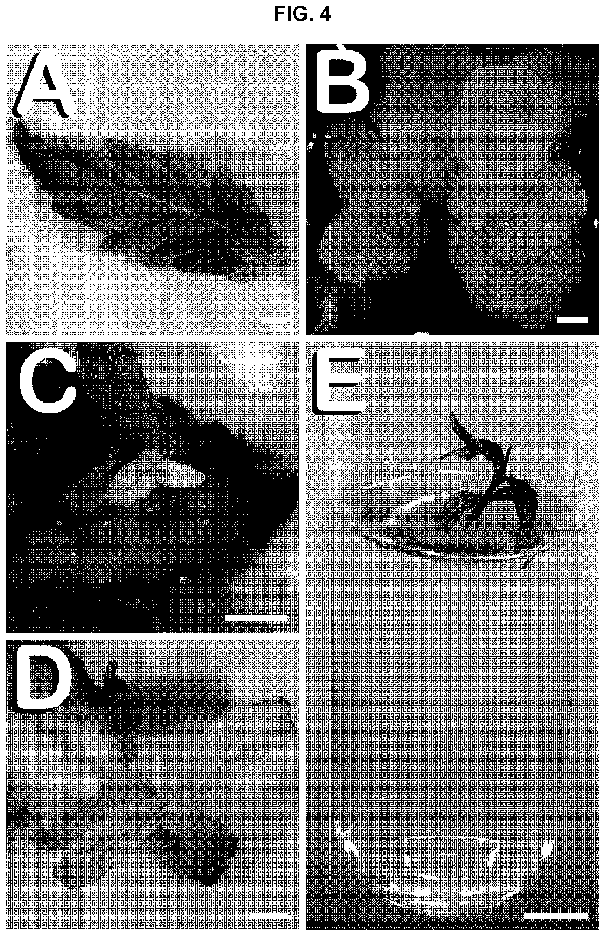

Figure 4: Direct *in vitro* shoot organogenesis from true leaves of *C. sativa.* The different developmental stages are described as follows: Newly dissected leaf from a seven-days-old hemp seedling (A). Non-responding leaf after two weeks in culture, where prominent non-organogenic callus development can be observed (B). Formation of one primordium from leaf-petiole transition zone one week after culture initiation (C). Two-week-old plantlet of approximately one centimeter in height ready for subculture (D). Leaf derived plant individually grown in a glass-tube 21 days after culture initiation (E). Scale bars (A-D): 1 mm. Scale bar (E): 6 mm.

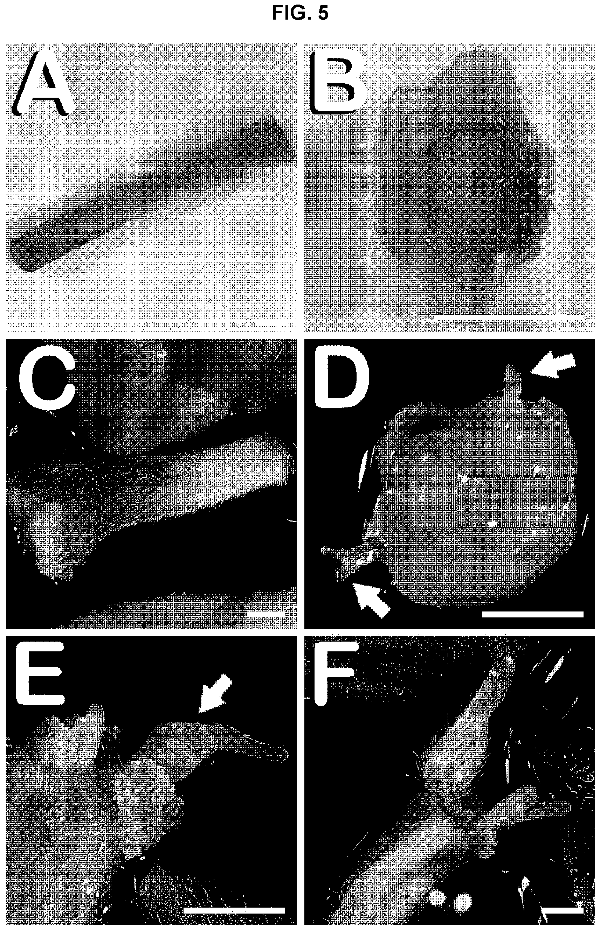

Figure 5: Direct *in vitro* shoot organogenesis from epicotyls of *C. sativa.* The different developmental stages are described as follows: Newly dissected epicotyl from a C. *sativa* seedling with four pairs of true leaves (A). Transverse section of newly dissected hemp epicotyl (B). Non-responding epicotyl after two weeks in culture showing tissue oxidation on the top of the explant (C). Two torpedo embryos (arrows) arising from opposite sides of the epicotyl segment after four days of *in vitro* culture (D). Detail of a newly developed shoot (arrow) eight days after explant inoculation (E). Two epicotyl derived regenerants 10 days after explant culture (F). Scale bars (A-F): 1 mm.

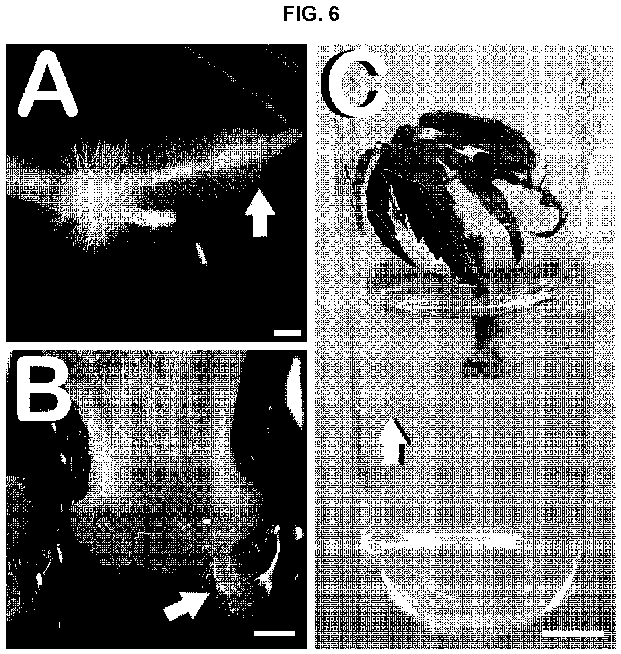

Figure 6: Rooting of explants and spontaneous rooting of hypocotyl derived plants of *C. sativa.* Vigorous root with radicular hairs emerging from the basal zone of the hypocotyl two weeks after culture initiation (arrow) (A). Small root with root hairs arising from the lower part of the cotyledon after 14 days of *in vitro* culture (arrow) (B). Spontaneously rooted hypocotyl derived plant after 28 days of culture initiation with a long root (arrow) (C). Scale bars (A-B): 1 mm. Scale bar (C): 6 mm.

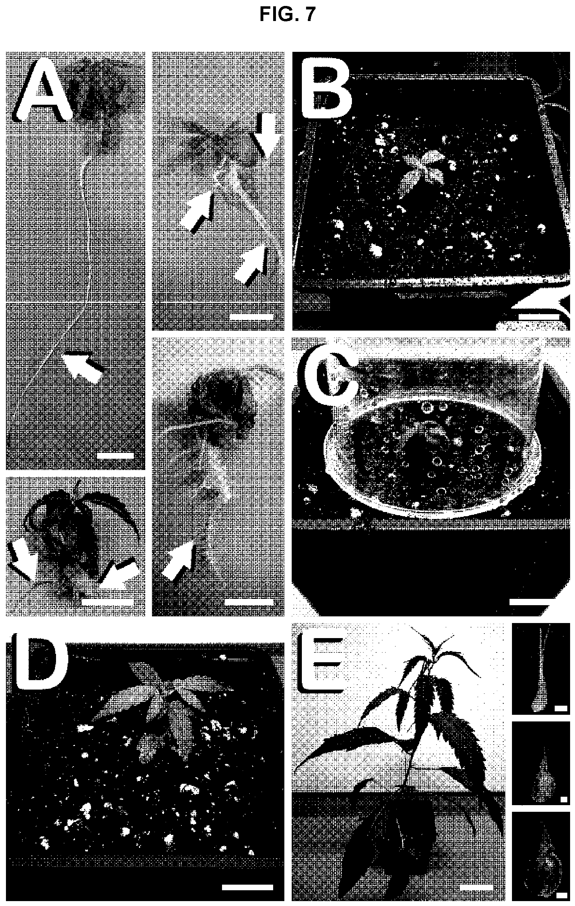

Figure 7: Acclimatization process of hypocotyl derived plants in *C. sativa.* The different developmental stages are described as follows: Radicular system of hypocotyl derived plants spontaneously rooted 28 days after culture initiation, where different root morphogenesis patterns can be observed (arrows) (A). Small plant just after being transplanted to pots (2 L) with fertilized commercial substrate (B). Plastic vessel covering the *in vitro* regenerated plant in order to avoid desiccation (C). Hypocotyl derived plant exposed to the environmental humidity six weeks after culture initiation (D). Female hypocotyl derived hemp plant showing sexual functionality eight weeks after *in vitro* explant inoculation (insets illustrates, from top to bottom, unfertilized female flower, fertilized female flower during seed formation and mature seed final development) (E). Scale bars (A-D): 12 mm. Scale bar (E): 60 mm. Scale bars of insets (E): 1 mm.

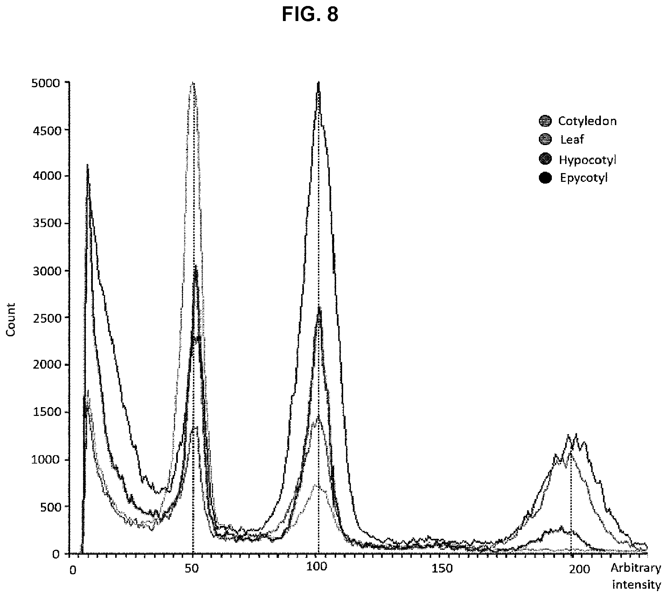

Figure 8: Flow cytometry histogram illustrating polysomatic pattern in cotyledons (blue), first pair of true leaves (green), hypocotyls (red), and epicotyls (black) from *C. sativa.* var. Finola. The x-axis represents a fluorescence intensity level proportional to the nuclear DNA content. The peak located at the value 50 corresponds to the diploid nuclei in phase G1, the peak located at the value 100 corresponds to the sum of the diploid nuclei in phase G2 and the tetraploid nuclei in phase G1, while the one at the value 200 represents tetraploid nuclei in G2 phase. The y-axis indicates the number of nuclei analyzed.

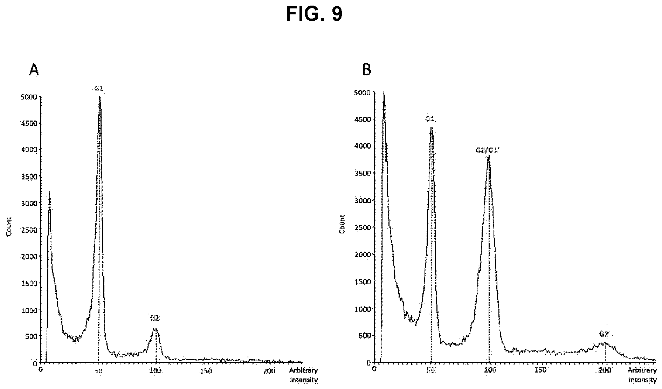

Figure 9: Nuclear DNA histogram patterns of diploid (A) and mixoploid (B) *in vitro* regenerated plants of *C. sativa* analyzed by flow cytometry. The x-axis represents a fluorescence intensity level proportional to the nuclear DNA content. The peak located at the value 50 corresponds to the diploid nuclei in phase G1, the peak located at the value 100 corresponds to the sum of the diploid nuclei in phase G2 and the tetraploid nuclei in phase G1, while the one at the value 200 represents tetraploid nuclei in G2 phase. The y-axis indicates the number of nuclei analyzed.

### DETAILED DESCRIPTION OF THE INVENTION

Plant regeneration involves the *in vitro* culture and aseptically growth of cells, tissues, and organs under defined physical and chemical conditions. Regeneration has long been known to occur in plants. In plants, non-differentiated cells are able to regenerate into the full array of organs and/or tissues under appropriate culture conditions. Regeneration can involve direct or indirect organogenesis. In direct regeneration, *in vitro* organs are directly induced from explant tissues; in indirect regeneration, a de *novo* organ is typically formed from an intermediate tissue, the callus. Plant calli are undifferentiated structures that can give rise to new tissues. Plant leaves, shoots, roots, and embryos can variously be elicited from a growing callus by treating it with different ratios of hormones. However, *in vitro* regeneration of a plant from a callus is often associated with loss of genetic fidelity of the regenerants with respect to the donor plant.

It has been known from the prior art a method of *in vitro* regeneration of *Cannabis sativa* L. from explant hypocotyl (Movahedi *et al.*, 2016a). However, such method is not a direct regeneration method, but an indirect *in vitro* regeneration method which probably due to the addition of plant growth regulators in the culture medium, comprises a callus formation phase taking place prior to shoot organogenesis. The formation of tissue or a new plant from the intermediate tissue (callus), seriously compromises the genetic fidelity of regenerants with respect to the donor plant.
To the contrary, the present invention concerns a direct *in vitro* regeneration method of *Cannabis* plants which avoids formation of a callus phase and by avoiding this phase, does not compromise the genetic fidelity of the regenerants obtained with respect to the donor plant.
For that purpose, the method advantageously uses apical meristems, stem nodes, internodes, cotyledons, true leaves, and preferably, hypocotyls and/or epicotyls from a plant with a phenological growth stage coded from 00 to 99 according to BBCH-scale of Mishchenko *et al.* (2017), preferably seven-days-old seedlings are used as donor *Cannabis* plants. By the method of the present invention, *Cannabis* plants are *in vitro* regenerated from said specific explants at a high rate of shoot organogenesis and/or embryogenesis. In other words, a high rate of shoot organogenesis and/or embryogenesis means that at least 22% of the explants give rise to *Cannabis sativa* L. *in vitro* plant regeneration. Preferably, regeneration occurs in at least 40% of cultured explants, even more preferably, regeneration occurs in at least 60% of cultured hypocotyl explants.
In a preferred embodiment, when apical meristem, stem node, internode, true leaf, hypocotyl and/or epicotyl explants are cultured according to the present invention's method, it is not even required addition of hormones, growth factors, plant growth regulators or any other substance or composition which induces or improves the formation or development of any multicellular structure or organ in the plant (e.g. embryo, shoot and/or root) to the culture medium.

As has been reported previously, one of the most important factors in adventitious organ formation is the endogenous auxin:cytokinin balance and not the amount of auxin or cytokinin added in the culture medium (Tanimoto and Harada, 1984).
Cytokinins are produced predominantly in the root meristem and auxins are synthetized in the shoot meristem, and both types of phytohormones can migrate from roots and shoots to their action site through phloem and xylem (Beck, 1996). It is believed that segmentation of both shoot and root meristems as a result of hypocotyl or epicotyl dissection could modify the endogenous hormonal interaction between auxins and cytokinins, leading to an appropriate environment for shoot and/or root organogenesis development in hypocotyls and epicotyls.

The present invention is also based in the findings that hypocotyl and epicotyl derived plants can root spontaneously in a hormone-free culture medium, which comprises no hormones, no growth factors and/or any plant growth regulator and/or any other substance or composition eliciting a plant growth regulator effect, thus being able to completely be acclimatized in only six weeks.

It is also believed that the same reasoning described above explains the fact that after shoot development in the top of the hypocotyl or epicotyl, auxins produced endogenously in the shoot meristem could promote the spontaneous rooting of *in vitro* regenerants, representing an added advantage of the method according to the present invention, since a separate auxin containing medium is not required for root induction. Advantageously, the author of the present invention has found that hypocotyls cultured in a medium free of plant growth regulators reached the third highest shoot induction rate of the evaluated media without presenting significant differences with the other two media with better percentages of shoot organogenesis (see table 5 below). In the case of *Cannabis* epicotyl *in vitro* culture, hormone-free medium reached the highest shoot induction rate of the evaluated media (see table 3 below). In addition, thereto, when hypocotyls and/or epicotyls were used as explants in a hormone-free medium according to the method described in the present invention, plants spontaneously rooted and thereafter were completely acclimatized in just six weeks.

It is also believed that pericycle cells adjacent to xylem poles could be the origin of *in vitro* direct regenerated plants of *Cannabis* according to the present invention.

In order to infer the possible origin of *in vitro* regenerants from cotyledons, hypocotyls, epicotyls and true leaves coming from seven-days-old seedlings, the authors of the present invention have examined transversal sections of hypocotyls and epicotyls and identified pith, cortex and epidermis. These observations are consistent with those documented in several prior art references. As presented by Behr *et al.* (2016), cross-sections of cannabis hypocotyls six and nine days after sowing are coincident with the results obtained by the author of the present invention, since epidermis, cortex and pith can be easily differentiated and their respective anatomy is also concurrent with the present findings.
The fact that the two primordia emerged from the top of hypocotyls and the lower section of epicotyls were always distributed in the periphery of the organ and aligned one in front of the other, led the author of the present invention to hypothesize that regenerated plants originated always from the same type of cells. In a former work by Miller (1959), hop hypocotyl cross-sections drawings detailed the connection between root and cotyledons of the seedling, describing not only the same regions than in the hypocotyl transversal section of the present invention's findings, but also two protoxylem poles situated in a peripheral position and distributed in opposite sides, whose location strongly resembles the regeneration area of hypocotyl and epicotyl derived meristems in the experiments of the present invention. Furthermore, this prior state of the art also describes how only one protoxylem pole is located in the median strand of the base of each cotyledon. The fact that in the present invention's study plant regeneration from cotyledons always was located in the central region of the basal zone of the explant, supports the hypothesis that cotyledon, hypocotyl and epicotyl derived plant regeneration in *C. sativa* originates from pericycle cells adjacent to xylem poles. It should be noted that hop (*Humus lupulus* L.) is the only species together with *C. sativa* belonging to Cannabaceae family, so it should be considered as an ideal candidate to compare with *Cannabis sativa* L. With respect to the present invention's observations concerning true leaf derived plant regeneration, although in this study only five plants were regenerated from leaves, it is remarkably how all of them were originated from leaf-petiole transition zone. The fact that vascularization also takes place in petioles, as it does also on stems, and that leaf regenerated plants always emerged from petioles, could fit with the present invention's hypothesis concerning pericycle-derived *in vitro* shoot organogenesis in this species. This extends the scope of the present invention's protocol towards micropropagation purposes, adding the possibility to produce multiple clones genetically identical to the mature elite plants already selected from which they could be derived. In this respect, it is important to emphasize how cotyledon, hypocotyl, true leaf and epicotyl derived regenerants, while were maintained in glass-tubes, continued producing multiple shoots even six months after culture initiation.
It has been described in the state of the art how pericycle cells encircling the xylem pole are considered as an extended meristem which retain the capacity to undergo asymmetric cell division even when other cells have differentiated, and that some pericycle cells surrounded by differentiated cells can still become programmed to begin to proliferate, thus leading to the initiation of a new organ (Beeckman and De Smet, 2014). These findings explain how in the present invention, direct *in vitro* plant regeneration always developed directly with no need of cell dedifferentiation. It is believed that the implication of vascular traces on the regenerative capacity of the evaluated explants could explain the different shoot organogenesis processes according to the present invention.

The present invention is also based on the findings that polysomaty is present in cotyledons, epicotyls and hypocotyls of *C. sativa* seedlings.
The term polysomaty refers to the condition of those cells in the somatic tissues of a plant which contain multiples of the typical chromosome number (Ervin, 1941). Its role as one of the most crucial pathways in introducing speciation and broadening biodiversity, especially in the plant kingdom, has been highlighted by the authors skilled in the art (Van Hieu, 2019).
Endomitosis or endoreduplication are described as possible causes that may lead to polysomaty (D'Amato, 1964; Bubner *et a*/*.*, 2006), whose occurrence is related with growth and differentiation of tissues. It has also been described how plant tissues frequently contain a proportion of endopolyploid cells (Ramsay and Kumar, 1990, and references therein) and how portions of the plant such as storage organs and vessels often contain polyploid cells (Adelberg *et al.*, 1993). Concerning polysomaty in *C. sativa,* it should be noted how it was first described in root meristems of the species by Litardière (1925) and how it is known that the doubled number of chromosomes in root meristems coming from *C. sativa* resulted from two successive cleavages of each chromosome during the prophases (Langlet, 1927). Other authors proposed nuclear fusion as the cause of the polysomatic condition described in *C. sativa* roots (Breslavetz, 1926, 1932). The author of the present invention has analyzed the ploidy level of cotyledons, hypocotyls, epicotyls and true leaves coming from seven-days-old seedlings of *C. sativa* by means of flow cytometry and have described for the first time polysomaty in cotyledons, epicotyls and hypocotyls of *C. sativa.* In light of these results, while leaves preserved the diploid pattern typical of the species, cotyledons, epicotyls and hypocotyls displayed a polysomatic pattern containing diploid and tetraploid cells, and therefore should be considered as mixoploid organs. These findings concerning polysomaty found in cotyledons, epicotyls and hypocotyls of *C. sativa,* open new applications of the method of the invention, such as the development of polyploids through *in vitro* direct plant regeneration from these organs.

The present invention is also based on the findings that mixoploid plants can be regenerated after *in vitro* culture of hypocotyls, epicotyls and cotyledons coming from seedlings of *Cannabis.*
Polyploids are associated with enlarged organ sizes, increased biomass yield, phytochemical content and metabolic products, enhanced ability to adapt to biotic and abiotic stresses, and with changes on gene regulation (Van Hieu, 2019). Additionally, development of polyploid plants, in particular tetraploids, could be useful in plant breeding for development of triploid varieties with seedless fruits. Since polyploid nuclei may sometimes be the progenitors of a cell generation, giving rise to a patch of polyploid tissues (D'Amato, 1952) and after being aware of polysomaty in cotyledons, epicotyls and hypocotyls of *C. sativa,* the author of the present invention evaluated the ploidy level of *in vitro* regenerants. In this respect, no significant differences were detected between explants in terms of polyploidization of regenerated plants and it has been described how cotyledon, epicotyl and hypocotyl are the only explants capable to generate mixoploid plants. It should be noted how polyploidization uses to be associated with enhanced levels of secondary metabolites in a large number of species (lannicelli *et a*/*.*, 2019).
Polyploidization in *C. sativa* has always been induced by treating apical meristems with chemical microtubule disruptors with a high toxicity grade (Mansouri and Bagheri, 2017; Parsons *et a*/*.*, 2019), for which polyploid plants often revert back to the diploid condition (Clarke, 1981), forcing to test the ploidy level of polyploid plants throughout generations.
In conclusion, due the high regenerative capacity of hypocotyls and epicotyls and that only hypocotyl and epicotyl-derived *in vitro* regenerants have been able to spontaneously rooting, together with the absence of significant differences between media with the best shoot induction rates with respect to number of shoots per responding explant and between cotyledon, hypocotyl and epicotyl derived plants in terms of polyploidization, the present invention contemplates as a preferred embodiment of the invention the culture of hypocotyls and epicotyls in hormone-free medium without chemical microtubule disruptors as the most suitable of the treatments evaluated in this study in order to obtain polyploid *Cannabis* plants. The present invention's method for *in vitro* direct regeneration of *Cannabis* plants has important connotations in exploitation of contemporary plant breeding techniques like genome editing (e.g., by using CRISPR/Cas gene edition) or mutagenesis, being also useful for micropropagation and for the development of polyploid varieties with enhanced levels of cannabinoids without using toxic chemical inductors.

The method for *in vitro* direct regeneration of a polyploid *Cannabis* plant comprises the following steps:
a) Collecting seeds of a donor *Cannabis* plant;
b) Surface sterilization of the seeds with ethanol, bleach, mercuric chloride or other chemical or physical disinfectant agent. In a preferred embodiment, seeds are surface sterilized in 75% (v/v) ethanol during two minutes and 30 seconds, followed by immersion in 30 g/L of NaClO with 0.1% (v/v) of Tween 20 during 25 minutes, being finally washed three times (1 minute, 4 minutes and 5 minutes) in autoclaved deionized water;
c) Germination of the seeds in a Petri dish containing culture medium with plant growth regulators. In a preferred embodiment, the culture medium is composed of ½ MS basal salts and vitamins (Murashige and Skoog, 1962) + 1.5% (w/v) sucrose + 3.5 g/L Gelrite® with a pH value of 5.8. In some embodiments of the present invention the culture medium is hormone free.
d) Dissection of apical meristems, stem nodes, internodes, cotyledons, true leaves, hypocotyls and/or epicotyls comprising pericycle cells adjacent to xylem poles or meristematic cells from a plant in a phenological growth stage coded from 00 to 99 according to BBCH-scale of Mishchenko *et al.* (2017), more preferably, from seven-days-old *Cannabis* seedlings. In some embodiments, hypocotyls or epicotyls are dissected from seedlings at a phenological growth stage coded by number 11 of BBCH-scale for *C. sativa* (Mishchenko *et al.*, 2017).
e) Culturing of the explants in a culture media under controlled conditions of temperature and relative humidity during 2 - 3 weeks. In a preferred embodiment, explants are cultured at 22°C ±1°C and 60% ±1% relative humidity with a photoperiod of 16 hours of light and 8 hours of dark; In a preferred embodiment, plant growth regulators and/or hormones are absent in the culture medium. In some preferred embodiments, polyploid plants are regenerated from cotyledon, hypocotyl and/or epicolyl explants without using any chemical microtubule disruptor with a high toxicity grade in the culture medium. In some other embodiments, apical meristems, stem nodes, internodes, cotyledons, true leaves, hypocotyls and/or epicotyls comprising pericycle cells adjacent to xylem poles or meristematic cells are cultured in the presence of chemical microtubule disruptors and/or chemical mutagens.
f) Selection of embryos, shoots and/or shoots with roots;
g) Sub-culturing the specimens of f) individually to glass-tubes or other containers of different volumes with the culture media used in step e) until spontaneously rooted plants are generated or until spontaneously rooted plants develop enough to start the acclimatization process;
h) Transplanting spontaneously rooted plants in pots with fertilized substrate and acclimatizing as needed;

### Culture medium used in steps c), e) and g)

The present invention describes semi-solid or liquid media including, but not limited to macronutrients like CaCl₂, Ca(NO₃)₂ 4H₂O, MgSO₄, NaH₂PO₄, (NH₄)₂SO₄, NH₄NO₃, KNO₃, KH₂PO₄ and K₂SO₄, in concentrations ranging, but not limited to between 50 and 5,000 mg/L, including, but not limited to micronutrients like CoCl₂ 6H₂O, CuSO₄ 5H₂O, FeNaEDTA, H₃BO₃, KI, MnSO₄ H₂O, Na₂MoO₄ 2H₂O and ZnSO₄ 7H₂O, in concentrations ranging, but not limited to between 0.001 and 40 mg/L, including, but not limited to vitamins like glycine, myo-inositol, nicotinic acid, pyridoxine HCI, thiamine HCI, biotin, D(+)-biotine, folic acid, L-glutamine, gluthatione (reduced) and L-serine in concentrations ranging, but not limited to between 0.01 and 1,000 mg/L, including, but not limited to carbon sources like sucrose, D-fructose, D-galactose, D-glucose monohydrate, lactose monohydrate, maltose monohydrate, D-mannose, D-mannitol or D-sorbitol, including, but not limited to concentrations ranging from 1 g/L to 300 g/L, with or without gelling agents including, but not limited to plant agar, Daishin agar or Gelrite®, including, but not limited to concentrations ranging from 0 g/L to 10 g/L, with or without active charcoal, including, but not limited to concentrations ranging from 0 to 100 g/L, with or without plant growth regulators including, but not limited to 6-Benzylaminopurine (6-BAP), 6-Benzylaminopurine Riboside (N6-Benzyladenosine), 6-(3-hydroxybenzylamino)purine (Meta-Topoline), N-(2-chloro-4-pyridyl)-N-phenylurea (4-CPPU), 2-isopentenyladenine (2iP), 6-(Furfurylamino)purine (Kinetin), 6-(4-Hydroxy-3-methylbut-2-enylamino)purine (Zeatin, trans-isomer), 9-(β-D-Ribofuranosyl)-trans-zeatin (Zeatin Riboside, trans-isomer), 1-Phenyl-3-(1,2,3,-thiadiazol-5-yl)-urea (Thidiazuron), 3,6-dichloro-2-methoxybenzoic acid (DICAMBA), 2,4-Dichlorophenoxyacetic Acid (2,4-D), Indole-3-Butyric Acid (IBA), Indole-3-Acetic Acid (IAA), α-Naphtalene Acetic Acid (NAA), 4-amino-3,5,6-trichloropicolinic acid (Picloram), Phenylacetic acid (PAA), Gibberellin A3 (GA3), Gibberellin A4 (GA4), Gibberellin A7 (GA7), Abcisic Acid (S-ABA), p-Chlorophenoxyisobutyric acid (PCIB) or 2,3,5-Triiodobenzoic Acid (TIBA), including, but not limited to concentrations ranging from 0 mg/L to 1,000 mg/L and cytokinin:auxin ratios ranging from 1:1 to 1,000:1, with or without chemical microtubule disruptors including, but not limited to colchicine, amiprophos methyl (APM) or oryzalin added to the culture medium during a period of time including, but not limited to between 1 and 168 hours in concentrations ranging, but not limited to between 1 and 2000 mg/L, with or without alkylating agents including, but not limited to ethyl methanesulphonate (EMS), ethyl nitroso urethane (ENU) or methylnitrosourea (MNU) and/or non-alkylating agents including, but not limited to sodium azide (NaN₃), nitrous acid (HNO₂) or nitric oxide (NO), added to the culture medium during a period of time including, but not limited to between 1 and 72 hours in concentrations ranging, but not limited to between 0.1 and 200 mg/L.

In some embodiments, hypocotyls or epicotyls are preferably cultured in medium without plant growth regulators or hormones due spontaneous rooting of around 20% of *in vitro* regenerated plants.

In some embodiments, pH of the media is adjusted to a value between 4.0 and 8.0.

In some embodiments, any medium, or combinations thereof, of the present disclosure may be utilized for the *in vitro* culture of *Cannabis sativa L.* explants in plastic or glass tubes, vessels or containers of multiple volumes.

The present invention includes a unique culture in which *in vitro* plants are regenerated and rooted in the same step or multiple and sequential subcultures of the original explant or *in vitro* regenerated plantlets in a different container with the same or a different medium (e.g. culturing the original explant or *in vitro* regenerated plants in a different container with the same or a different medium for promoting growth of regenerated shoots, continuous, exponential and unlimited regeneration of shoots or the induction of rooting of *in vitro* regenerated plantlets).

### EXAMPLES

### Example 1: Plant material and growth conditions

Seeds from monoecious *C. sativa* short-day varieties Ferimon, Felina32, Fedora17 and USO31, together with seeds from dioecious neutral-day variety Finola were surface sterilized in 75% (v/v) ethanol during two minutes and 30 seconds, followed by immersion in 30 g/L of NaClO with 0.1% (v/v) of Tween 20 during 25 minutes, and finally washed three times in autoclaved deionized water. Once sterilized, seeds were germinated in 9 cm diameter Petri dishes containing previously autoclaved germination medium whose composition was ½ MS basal salts and vitamins (Murashige and Skoog, 1962) + 1.5% (w/v) sucrose + 3.5 g/L Gelrite® with a pH value of 5.8. After germination, explants (cotyledons, hypocotyls, leaf and epicotyls) dissected from seven-days-old seedlings, which is equivalent to the phenological growth stage coded in this species by number 11 in BBCH-scale (Mishchenko *et a*/*.*, 2017), were cultured in different media described in Table 1 below.
The culture medium is composed of ½ MS basal salts and vitamins (Murashige and Skoog, 1962) + 1.5% (w/v) sucrose + 3.5 g/L Gelrite®. The different culture media referred in Table 1 (see below) contain such medium composition without plant growth regulators (medium 0) and with different plant growth regulators (media 1 to 9).

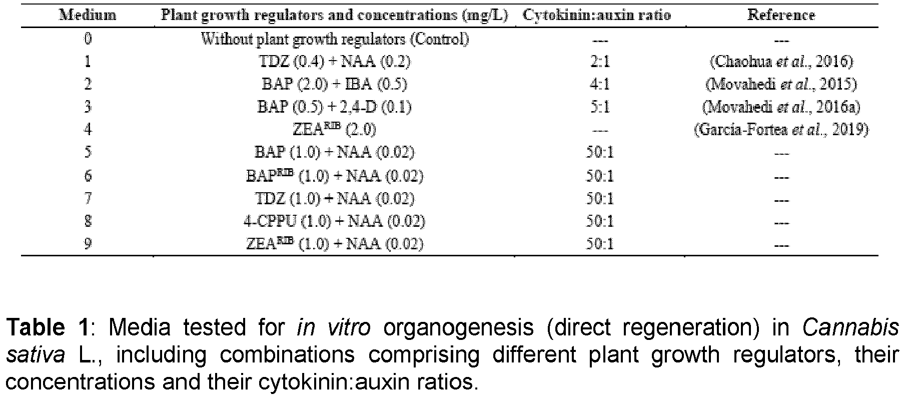

Seedlings and explants were grown under controlled conditions at 22°C ±1°C and 60% ±1% relative humidity. Photoperiod consisted of 16 hours of light and 8 hours of dark. Light was provided by LED tubes of 18W with a color temperature of 6,000K, which was translated in 6,010 lux and 90.1 µmol m⁻² s⁻¹. Explants producing shoots and roots, and number of shoots developed on each of responding explants were counted periodically during two weeks of culture. After that time, *in vitro* regenerants were subcultured individually to glass-tubes of 2.5 cm of diameter and 15 cm long, containing the same medium in which shoots were generated.
When roots were visible, spontaneously-rooted plants were cultured in pots (2 L) with fertilized commercial substrate with a pH value of 6 and a conductivity of 1 mS/cm. Previously, gelled medium was carefully washed from roots. After transplant and during the whole process of acclimatization, the substrate was maintained slightly moist and, twice per day, regenerants received foliar pulverization with water. To avoid desiccation, the small plants were covered with plastic vessels and were progressively exposed to the environmental humidity. Until complete acclimatization, plants were grown under identical conditions of temperature, photoperiod and light as described above.

### Example 2: In vitro shoot organogenesis experiments

In order to promote *in vitro* shoot organogenesis in *C. sativa,* cotyledons, hypocotyls, true leaves and epicotyls dissected from seven-days-old seedlings of the five short and neutral-day varieties, were cultured in culture medium with the same composition as described in Example 1, except for the addition of different plant growth regulators. As a part of this study, the author of the present invention aimed at evaluating with their own genotypes the efficiency of different *in vitro* shoot regeneration published protocols developed for *C. sativa.* Therefore, selected studies in which different explants, cytokinins and auxins and their ratios were successfully tested. In this way, the following medium used in a study regarding the regenerative capacity of cotyledons was tested through addition of thidiazuron (TDZ) and α-naphthaleneacetic acid (NAA) to the culture medium (Chaohua *et a*/*.*, 2016), one work concerning *in vitro* plant regeneration from cotyledons and epicotyls by means of 6-benzylaminopurine (BAP) and indole-3-butyric acid (IBA) (Movahedi *et al.*, 2015), and another one from leaves and hypocotyls through BAP and 2,4-dichlorophenoxyacetic acid (2,4-D) (Movahedi *et al.*, 2016a). Additionally, an effective and newly released protocol developed for eggplant, a species recalcitrant in terms of *in vitro* shoot regeneration, in which *in vitro* shoot organogenesis was achieved by use of zeatin riboside (ZEA^{RIB}) (García-Fortea *et al.*, 2019), was added to the present study.

Finally, as it is known that root and shoot development depends on cytokinin:auxin ratio and that high levels of cytokinin supports shoot formation (Skoog and Miller, 1957; Su *et al.*, 2011), the authors of the present invention tested the effect of a cytokinin:auxin ratio of 50:1 with different adenine and phenylurea derivatives like BAP, 6-benzylaminopurine riboside (BAP^{RIB}), TDZ, forchlorfenuron (4-CPPU) and ZEA^{RIB} plus NAA, an auxin commonly employed in protocols for *in vitro* regeneration of shoots (Plawuszewski *et al.*, 2006; Wielgus *et al.*, 2008; Lata *et a*/*.*, 2010; Chaohua *et al.*, 2016) and *in vitro* rooting of C. *sativa* (Lusarkiewicz-Jarzina *et al.*, 2005; Wang *et al.*, 2009; Movahedi *et al.*, 2016a; Parsons *et al.*, 2019). The different hormonal combinations present in the different shoot induction media evaluated in this work, are detailed in Table 1.

### Results: Effect of genotype, explant and medium on in vitro shoot organogenesis of C. sativa

The effect of explant on direct *in vitro* shoot organogenesis of *C. sativa* is shown in Table 2 (see below). As can be seen from these results, hypocotyl and epicotyl are shown to be preferred explants for the method of direct *in vitro* regeneration of *Cannabis* plants according to the present invention.

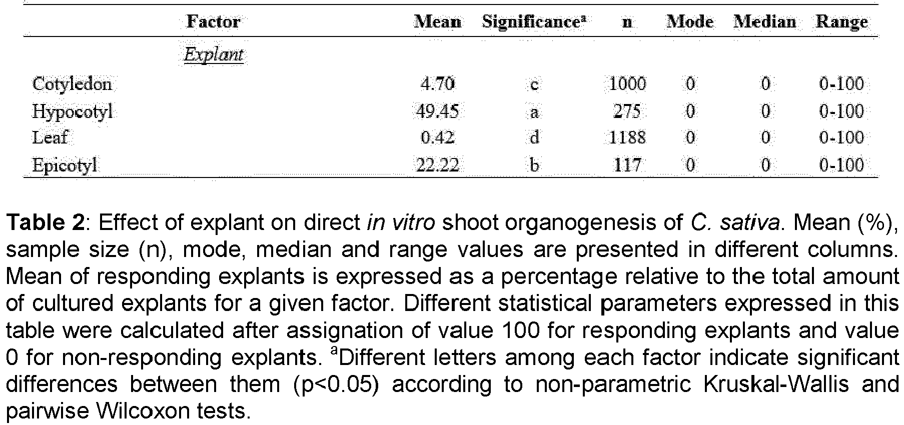

Shoot *in vitro* regeneration was observed in all *C. sativa* varieties, explant types and media tested, resulting in a total of 294 *in vitro* regenerated shoots, although significant differences (p<0.05) for the three main factors (variety, explant and culture medium) were observed. Regarding the factor explant and its effect on the percentage of explants developing shoots, significant differences were detected between cotyledons, hypocotyls, epicotyls and true leaves. On average, hypocotyl showed the best response in terms of direct plant regeneration, reaching 49.45% of explants with shoot formation, followed by epicotyl reaching 22,22%, cotyledon with 4.70% and true leaf with 0.42% (see Table 2 above).

Since true leaves displayed a weak capacity to induce *in vitro* direct shoot organogenesis, the authors of the present invention analyzed separately data from epicotyls (see Table 3 below), cotyledons (see Table 4 below) and hypocotyls (see Table 5 below).

When epicotyls were used as explant, they gave best performance in a culture medium free of plant growth regulators, as shown in Table 3 below.

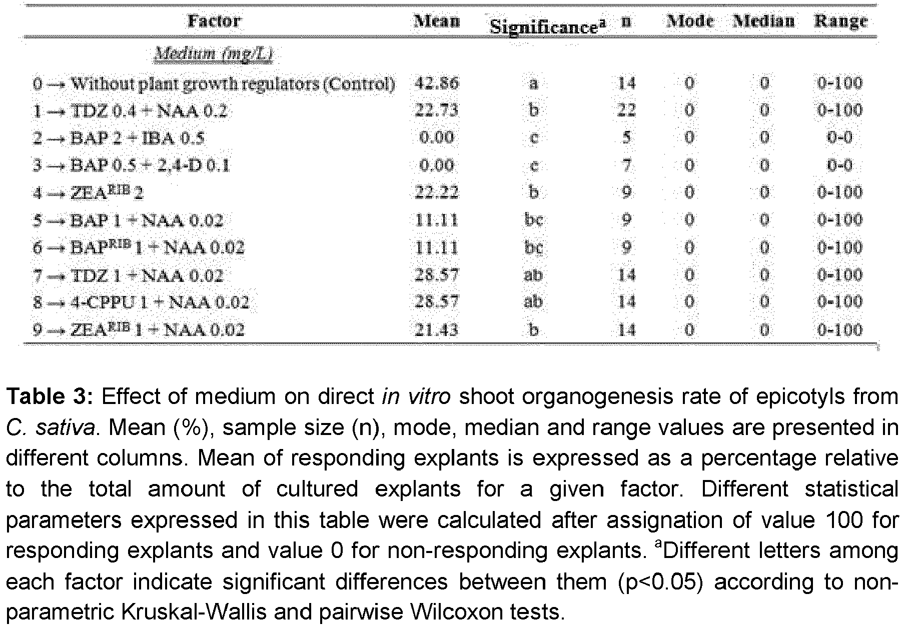

Regarding cotyledons, significant differences were observed among the different media tested. Medium 1 (TDZ 0.4 mg/L + NAA 0.2 mg/L) was the best, achieving the highest shoot induction rate with a 22.32% of responding explants (see Table 4 below). Medium 0 (without plant growth regulators) and number 9 (ZEA^{RIB} 1 mg/L + NAA 0.02 mg/L) were the worst treatments, without any explant showing response in terms of shoot organogenesis (see Table 4 below).

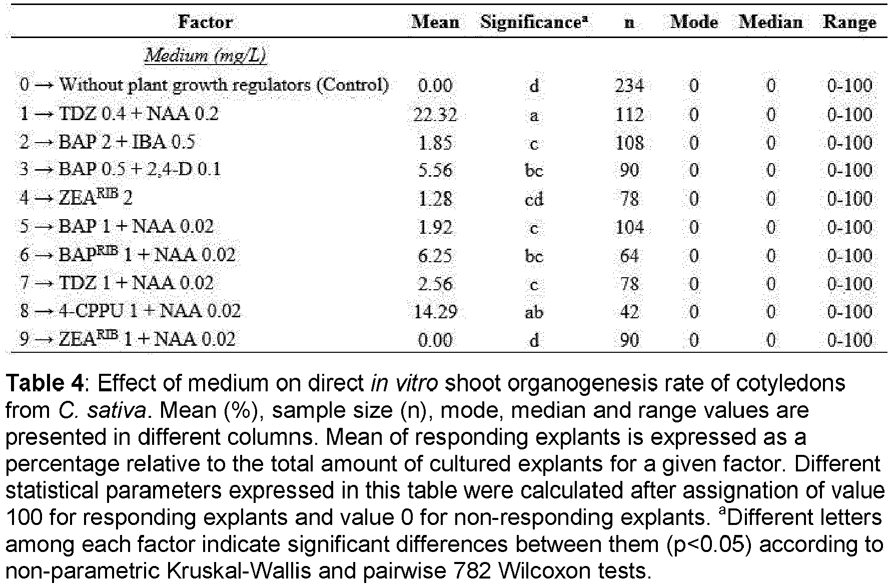

Concerning hypocotyl, significant differences were identified between the different varieties and media evaluated in this experiment. USO31 was the best variety evaluated, with 71.15% of its explants developing shoots (see Table 5 below), while Finola and Ferimon were the varieties with the lowest regeneration percentages with, respectively, 35.42% and 32.26% of its explants regenerating shoots (see Table 5 below). In relation to the effect of medium on shoot organogenesis, media number 4 (ZEA^{RIB} 2 mg/L) and number 9 (ZEA^{RIB} 1 mg/L + NAA 0.02 mg/L) resulted in the highest rate of shoot induction with 66.67% of responding explants, followed by medium 0 (without plant growth regulators) and medium 1 (TDZ 0.4 mg/L + NAA 0.2 mg/L) with, respectively, 61.54% and 54.17% of shoot organogenesis rate (see Table 5 below).

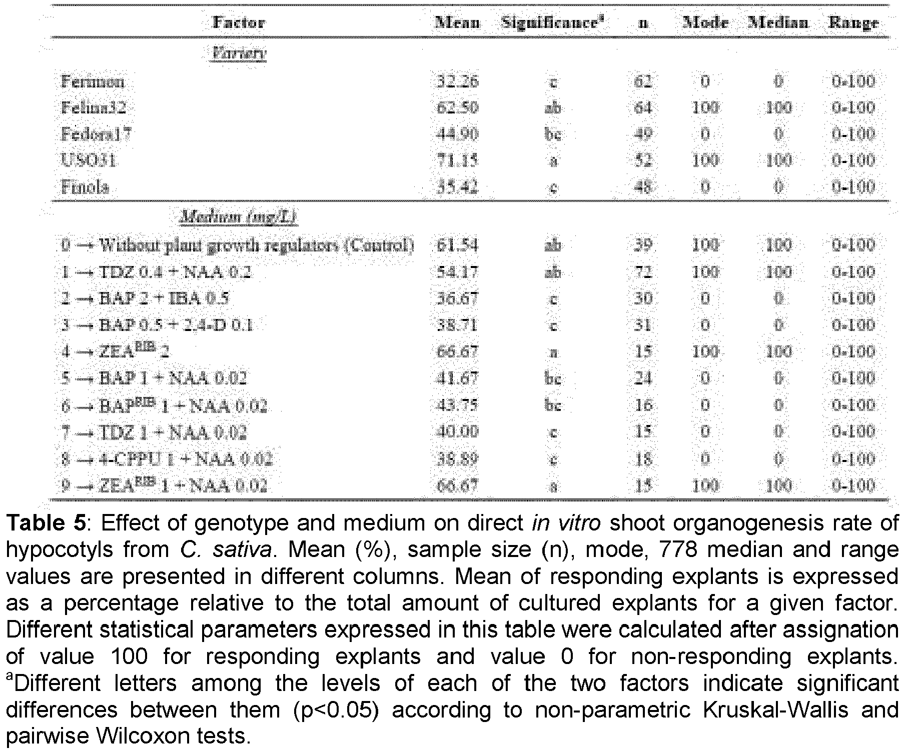

In addition, the number of shoots developed on each of the responding explants were statistically analyzed for the combinations of varieties and media with the best shoot induction rates identified in this study. In the case of cotyledons, as varieties USO31, Fedora17 and Ferimon and media 1 (TDZ 0.4 mg/L + NAA 0.2 mg/L) and 8 (4-CPPU 1 mg/L + NAA 0.02 mg/L) gave the best shoot induction rates, their number of shoots per responding explant were statistically compared (see Table 6 below). Although no significant differences were found between varieties and media in terms of number of shoots per responding cotyledon, Fedora17 showed the best results with 1.42 shoots per responding explant, while medium 1 (TDZ 0.4 mg/L + NAA 0.2 mg/L) reached 1.28 shoots per responding cotyledon (see Table 6 below).

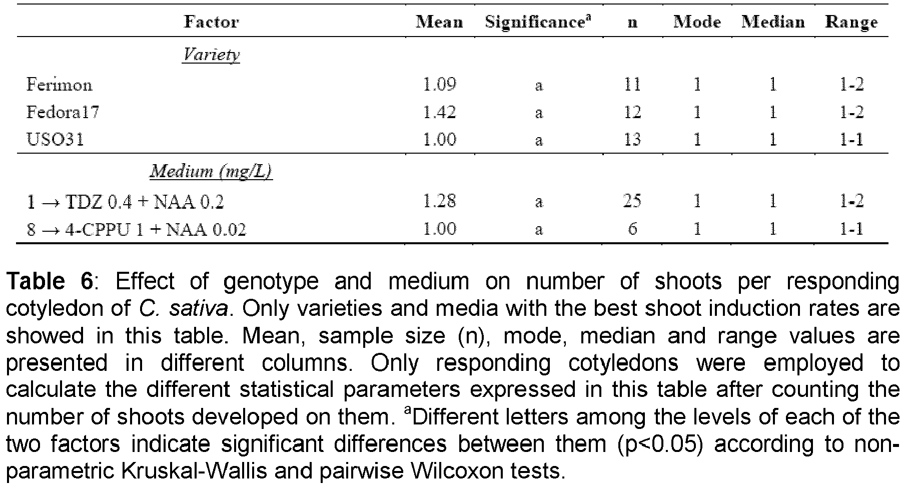

Regarding hypocotyls, since varieties USO31 and Felina32 and media 0 (without plant growth regulators), 1 (TDZ 0.4 mg/L + NAA 0.2 mg/L), 4 (ZEA^{RIB} 2 mg/L) and 9 (ZEA^{RIB} 1 mg/L + NAA 0.02 mg/L) attained the best shoot organogenesis rates, their number of shoots per responding explant were also statistically compared (see Table 7 below). Once again, USO31 exhibited the best response in terms of number of shoots per responding hypocotyl, reaching 1.70 shoots per responding explant (see Table 7 below). Furthermore, although no significant differences were found among media tested, medium 4 (ZEA^{RIB} 2 mg/L), closely followed by medium 0 (without plant growth regulators), were the best media evaluated in this experiment with, respectively, 1.60 and 1.54 shoots per responding hypocotyl (see Table 7 below).

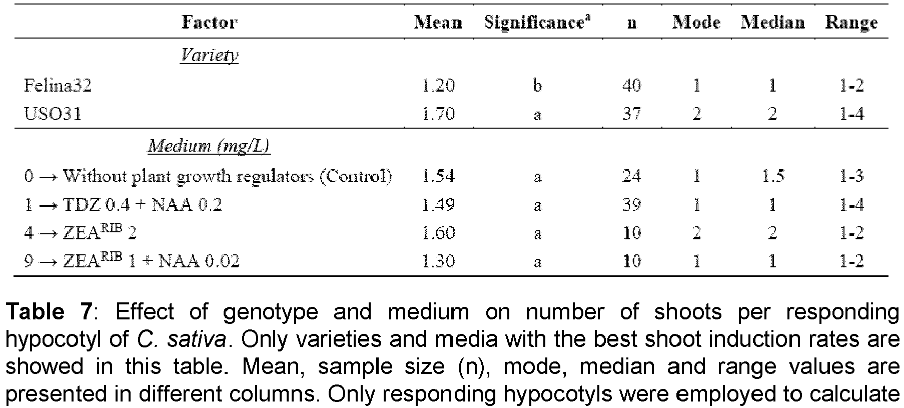

the different statistical parameters expressed in this table after counting the number of shoots developed on them. ^{a}Different letters among the levels of each of the two factors indicate significant differences between them (p<0.05) according to non-parametric Kruskal-Wallis and pairwise Wilcoxon tests.

Concerning epicotyls, since medium 0 (without plant growth regulators), medium 7 (TDZ 1 mg/L + NAA 0.02 mg/L) and medium 8 (4-CPPU 1 mg/L + NAA 0.02 mg/L) reached the highest percentages of responding explants, their data were employed to calculate the number of shoots per responding explant on each of them (see table 8 below). Although no significant differences were detected between the different media evaluated, medium 7 (TDZ 1 mg/L + NAA 0.02 mg/L) got the best results with 1.75 shoots per responding epicotyl (table 8).

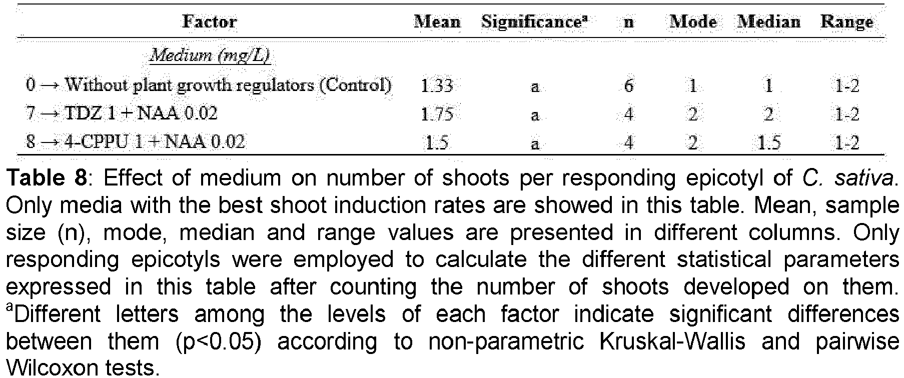

### Example 3: Developmental morphology of the in vitro regeneration process

The whole developmental process of *in vitro* shoot organogenesis was followed since germination of seeds to the acclimatization of plants. The time needed for each of the different developmental stages was registered. High resolution images of the different developmental stages were recorded with a stereozoom- microscope equipped with a digital camera.

In order to estimate the time needed to obtain and acclimatize *in vitro* regenerated *C. sativa* plants, the duration of each of the different stages throughout whole culture process was recorded. Additionally, all the developmental process from germination of seeds until acclimatization of *in vitro* regenerated plants was registered with images. First of all, seeds of the different varieties (Fig. 1A) were surface sterilized. Between 24 and 48 h after being cultured in germination medium, seeds started to germinate and the apical root meristem arose from the testa (Fig. 1B). Five days after *in vitro* sowing, seedlings liberated from the testa were visible while emerging (Fig. 1C). On the seventh day from seed plating, the first pair of true leaves were fully expanded (Fig. 1D). When seedlings arrived to this developmental stage, explants needed to continue the experiments were dissected.
At this stage of seedling development, cotyledons were easily dissected (Fig. 2A). Some cotyledons did not respond to any of the studied treatments. After two weeks of exposure to the culture medium, non-responding cotyledons acquired a brown coloration and, some of them, developed non-organogenic green calli (Fig. 2B), which were unable to regenerate shoots even after being maintained for one month in culture. On the other hand, the first steps of direct shoot organogenesis were rapidly visible on the basal zone of responding cotyledons. In this way, shoot primordia formation was centrally located at the proximal part of cotyledons after four days of *in vitro* culture (Fig. 2C). Two weeks after culture explant, vigorous regenerants arising from the proximal edge of cotyledons were observed (Fig. 2D). Once arrived to this regeneration stage, since regenerants reached approximately one centimeter in height and in order to avoid their contact with the Petri dish lid, *in vitro* regenerated shoots were subcultured individually in glass-tubes containing the same medium in which they were generated (Fig. 2E). A total of 47 cotyledons responded to the different treatments evaluated, generating a total amount of 54 shoots.
Alternatively, hypocotyls were cut from seven-days-old seedlings. Hypocotyls employed in this experiment measured approximately one centimeter in height (Fig. 3A). Transversal section of freshly dissected hypocotyls revealed its internal structure, with different tissue layers such as epidermis, cortex and pith (Fig. 3B). Non-responding hypocotyls showed oxidation signs on its top after two weeks of *in vitro* culture (arrow in Fig. 3C). On the other hand, responding explants exhibited different direct organogenesis patterns. Some of the hypocotyls generated only one primordium on the top of the explant which was originated from the central region of the section (Fig. 3D), and continued its development until becoming a robust plant after two weeks of culture (Fig. 3E). Other explants gave rise to a couple of primordia in the periphery of the organ and on opposite sides (arrows in Fig. 3F). In this last case, there were situations in which development between both primordia was asynchronous, while in other cases, growth and height of both regenerants was coincident, as can be observed in Fig. 3G. In the case illustrated by Fig. 3H, both *in vitro* regenerants reached approximately one centimeter in height two weeks after culture initiation. At this stage of organogenic development, shoots were detached from hypocotyls and individually cultured in glass-tubes containing the same medium in which they were generated (Fig. 3I). A total of 136 hypocotyls responded to the different treatments evaluated, producing a total amount of 196 shoots.

Additionally, the regenerative capacity of the first pair of true leaves from seven-days-old seedlings was also studied. For this, each leaf was carefully dissected (Fig. 4A) and cultured in the different media evaluated in this experiment. Two weeks after culture, non-responding leaves showed a prominent callus development (Fig. 4B). Even keeping these calli in culture for one month, plant regeneration never took place. Otherwise, when direct *in vitro* plant regeneration occurred, primordia arose always from the base of leaves, specifically from the petiole fragment attached to the leaf, as is the case of Fig. 4C, where a small plantlet arising from the leaf-petiole transition zone can be seen one week after culture. Shoot development continued until regenerated plants reached approximately one centimeter in height (Fig. 4D). Fourteen days after culture initiation, successful excision of shootlets was performed and regenerated plants were individually subcultured in glass-tubes containing the same medium in which they were generated (Fig. 4E). Only five leaves responded to any of the different treatments evaluated, generating a total amount of five shoots.

Finally, the different developmental stages of shoot organogenesis from epicotyls were carefully examined. Epicotyls were dissected from *C. sativa* seedlings with four pairs of true leaves (Fig. 5A). Transverse sections of epicotyl elucidated its internal layers, which were concurrent with those identified in hypocotyls (Fig. 5B). Non-responding epicotyls after two weeks in culture showed tissue oxidation on the top of the explant (Fig. 5C). As it occurred with hypocotyls, *in vitro* shoot regeneration from epicotyls was located in the periphery of the explant, with newly-formed shoots facing one to each other, as illustrated in Fig. 5D, where two torpedo embryos (arrows) arising from opposite sides of the epicotyl segment after four days of *in vitro* culture can be observed. After eight days of *in vitro* culture, newly developed shoots (arrow) showed a vigorous growth (Fig. 5E), while 10 days after explant culture, both epicotyl derived regenerants showed a synchronized development (Fig. 5F).

### Results: Rooting of explants and spontaneous rooting of hypocotyl-derived plants

Although the present study and its derived experiments were focused on *in vitro* shoot organogenesis, some of the cultured explants developed roots instead of shoots. Specifically, two weeks after culture initiation, 1.09% of cultured hypocotyls developed vigorous roots with root hairs on the lower zone of the explant, as illustrated in Fig. 6A.

The same phenomenon, also located on the proximal part of the explant, was observed in 0.1% of cultured cotyledons two weeks after explant culture (Fig. 6B). In another way, spontaneous rooting of *in vitro* hypocotyl-derived regenerants only took place in medium without plant growth regulators, where 17.94% of cultured hypocotyls developed shoots on its top and roots in its lower part. After 28 days of culture initiation, hypocotyl-derived plants spontaneously rooted were ready for start the acclimatization process (Fig. 6C).

After spontaneous rooting of *in vitro* regenerants, these plantlets were submitted to the acclimatization process. The first step consisted of carefully washing the remaining gellified medium from roots. After 28 days of *in vitro* culture, regenerants showed different root morphogenesis patterns as illustrated in Fig. 7A, where long, medium and short size roots can be visualized, together with a robust main root with a prominent development of secondary roots. Vigorous development of the radicular system guaranteed successful acclimatization of *in vitro* regenerated plants. At this point, regenerants were ready for transplant in small pots (2 L) with fertilized commercial substrate (Fig. 7B), although placement of transparent plastic vessels was necessary to retain humidity and avoid desiccation of plants (Fig. 7C). However, after one week of progressive exposition of regenerants to the environmental humidity, the acclimatization process ended and *in vitro* regenerated plants displayed a vigorous growth, as can be observed in Fig. 7D, where a healthy regenerant stands out six weeks after culture of hypocotyls. In order to verify the proper development of *in vitro* regenerants, acclimatized plants were grown during two additional weeks and were manually pollinated. As illustrated in Fig. 7E, *in vitro* regenerated plants showed sexual functionality eight weeks after *in vitro* explant inoculation, as can be deducted from the fact that female flowers developed viable seeds after manual pollination (insets in Fig. 7E). Following this protocol, 100% of *in vitro* regenerated plants spontaneously rooted were successfully acclimatized.

### Example 4: Determination of ploidy level of explants and in vitro regenerants

Ploidy level of cotyledons, hypocotyls, epicotyls and leaves from *in vitro* grown seven-days-old seedlings was evaluated to verify their polysomatic pattern. The four short-day varieties Ferimon, Felina32, Fedora17 and USO31, together with dioecious neutral-day variety Finola were analyzed in this experiment. Three seedlings coming from each variety were employed for this assay. On the other hand, young leaves from *in vitro* regenerated plants were also examined. Ploidy level of 38 *in vitro* regenerants (17 from cotyledons, 15 from hypocotyls, three from epicotyls and three from leaves) was determined. Cell nuclei of explants dissected were mechanically isolated. Sections of approximately 0.5 cm² were chopped with a razor blade in a 6 cm diameter glass Petri dish containing 0.5 ml lysis buffer LB01 (pH 7.5) (Dpooležel *et a*/*.*, 1989), and incubated for 5 minutes. Subsequently, the suspension containing nuclei and cell fragments was filtered using a 30 µm CellTrics filter (Sysmex, Sant Just Desvern, Spain). The nuclei in the filtrate were stained with CyStain UV Ploidy (Sysmex) and incubated for 5 minutes. The fluorescence intensity of the homogenate was measured using a CyFlow® Ploidy Analyser Sysmex Partec GmbH, analyzing at least 4,000 nuclei for each sample. Young leaves of diploid plants from all varieties studied were used as control. A diploid control peak was established at 50 points of the arbitrary intensity value of the fluorescence in the histogram. By comparison with this peak, the ploidy of the other tissues evaluated was checked.

The analysis of the ploidy level of freshly dissected cotyledons, hypocotyls, epicotyls and true leaves of seven-days-old seedlings of the five varieties evaluated revealed that only true-leaves (green) showed a diploid pattern, while cotyledons (blue), hypocotyls (red) and epicotyls (black) exhibited a mixoploid pattern (with diploid and tetraploid cells) (Fig. 8). All varieties evaluated in this experiment displayed the same polysomatic pattern for the different explants analyzed.

A total of 38 *in vitro* regenerated plants (17 from cotyledons, 15 from hypocotyls, three from epicotyls and three from leaves) were analyzed by means of flow cytometry at 28 days after tissue culture initiation. Only diploid and mixoploid plants (with diploid and tetraploid cells) were obtained. Differences in nuclear DNA histogram patterns between diploid (Fig. 9A) and mixoploid (Fig. 9B) plants are represented in a flow cytometry histogram (Fig. 9). As illustrated in Table 9 (see below), no significant differences were identified between cotyledon, hypocotyl and epicotyl-derived plants in terms of polyploidization of *in vitro* regenerants. Cotyledons, hypocotyls and epicotyls produced, respectively, 82.35%, 86.67% and 66.66% of diploid regenerants (Table 9). Regarding mixoploid regenerants, cotyledons, hypocotyls and epicotyls exhibited a significant capacity to generate them, with, respectively, 17.65%, 13.33% and 33.33% of mixoploid *in vitro* regenerated plants (Table 9).

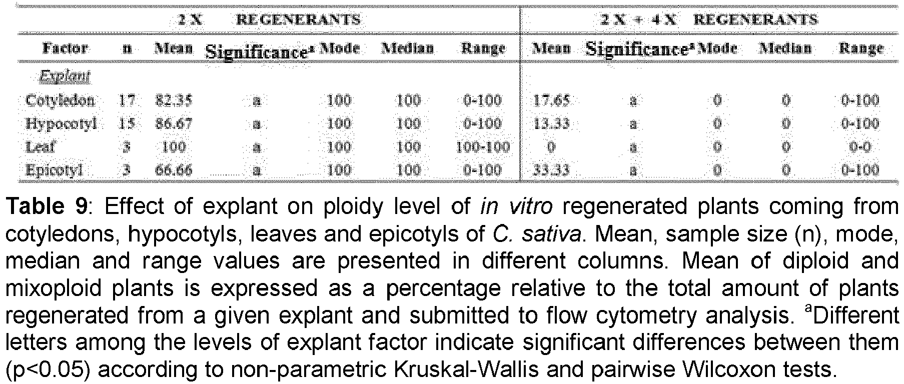

### REFERENCES

Adelberg, J. W., Rhodes, Bill B. and Skorupska, H. T. (1993). Generating tetraploid melons in tissue culture. Acta Hortic. 336, 373-380.
Andre, C. M., Hausman, J. F., and Guerriero, G. (2016). Cannabis sativa: The plant of the thousand and one molecules. Front. Plant Sci. 7. doi:10.3389/fpls.2016.00019.
Beck, E. H. (1996). Regulation of shoot/root ratio by cytokinins from roots in Urtica dioica: Opinion. Plant Soil 185, 3-12. doi:10.1007/bf02257560.
Beeckman, T., and De Smet, I. (2014). Pericycle. Curr. Biol. 24, R378-R379. doi:10.1016/j.cub.2014.03.031.
Behr, M., Legay, S., Žižková, E., Motyka, V., Dobrev, P. I., Hausman, J. F., et al. (2016). Studying secondary growth and bast fiber development: The hemp hypocotyl peeks behind the wall. Front. Plant Sci. 7. doi:10.3389/fpls.2016.01733.
Breslavetz, L. (1926). Polyploide Mitosen bei Cannabis sativa L. Ber. Deutsch. Bot. Ges. 44, 498-502.
Breslavetz, L. (1932). Polyploide Mitosen bei Cannabis sativa L. II. Ber. Deutsch. Bot. Ges. 17, 644-649.
Bubner, B., Gase, K., Berger, B., Link, D., and Baldwin, I. T. (2006). Occurrence of tetraploidy in Nicotiana attenuata plants after Agrobacterium-mediated transformation is genotype specific but independent of polysomaty of explant tissue. Plant Cell Rep. 25, 668-675. doi:10.1007/s00299-005-0111-4.
Cascio, M. G., Pertwee, R. G., and Marini, P. (2017). "The pharmacology and therapeutic potential 593 of plant cannabinoids," in Cannabis sativa L. - Botany and Biotechnology (Springer International Publishing), 207-225. doi:10.1007/978-3-319-54564-6_9.
Chaohua, C., Gonggu, Z., Lining, Z., Chunsheng, G., Qing, T., Jianhua, C., et al. (2016). A rapid shoot regeneration protocol from the cotyledons of hemp (Cannabis sativa L.). Ind. Crops 597 Prod. 83, 61-65. doi:10.1016/j.indcrop.2015.12.035.
Clarke, R. C. (1981). "The genetics and breeding of Cannabis," in Marijuana Botany: An Advanced Study: The Propagation and Breeding of Distinctive Cannabis, eds N. Hamel and D. Cross (Berkeley, CA: Ronin Publishing), 27-59.
D'Amato, F. (1952). Polyploidy in the differentiation and function of tissues and cells in plants. Caryologia, 4, 311-358. doi:10.1080/00087114.1952.10797544.
D'Amato, F. (1964). Endopolyploidy as a Factor in Plant Tissue Development. Caryologia 17, 41-52. doi:10.1080/00087114.1964.10796115.
Dpooležel, J., Binarová, P., and Lcretti, S. (1989). Analysis of Nuclear DNA content in plant cells by Flow cytometry. Biol. Plant. 31, 113-120. doi:10.1007/BF02907241.
Ervin, C. D. (1941). A study of polysomaty in Cucumis melo. Am. J. Bot. 28, 113-124.
Feeney, M., and Punja, Z. K. (2003). Tissue culture and Agrobacterium-mediated transformation of hemp (Cannabis sativa L.). Vitr. Cell. Dev. Biol. - Plant 39, 578-585. 621 doi:10.1079/IVP2003454.
Feeney, M., and Punja, Z. K. (2015). Hemp (Cannabis sativa L.). Methods Mol. Biol. 1224, 319- 329. doi:10.1007/978-1-4939-1658-0_25.
Feeney, M., and Punja, Z. K. (2017). "The role of Agrobacterium-mediated and other gene-transfer technologies in cannabis research and product development," in Cannabis sativa L. - Botany and Biotechnology (Springer International Publishing), 343-363. doi:10.1007/978-3-319-54564-6_16.
Garcia-Fortea, E., Lluch-Ruiz, A., Pineda-Chaza, B., García-Pérez, A., Bracho-Gil, J. P., Plazas, M., et al. (2019). Development of a new highly efficient organogenesis protocol in eggplant (Solanum melongena) and evaluation of the ploidy level. 17th Eucarpia Genet. Breed. capsicum eggplant*.*
Hemphill, J. K., Turner, J. C., and Mahlberg, P. G. (1978). Studies on growth and cannabinoid composition of callus derived from different strains of Cannabis sativa. Lloydia 41, 453-462.
Iannicelli, J., Guariniello, J., Tossi, V. E., Regalado, J. J., Di Ciaccio, L., van Baren, C. M., et al. (2019). The "polyploid effect" in the breeding of aromatic and medicinal species. Sci. Hortic. (Amsterdam). 260, 108854. doi:10.1016/j.scienta.2019.108854.
Langlet, O. (1927). Zur Kenntnis der polysomatischen Zellkerne im Wurzelmeristem. Sven. Bot. Tidskr. 21, 397-422.
Lata, H., Chandra, S., Khan, I. A., and Elsohly, M. A. (2010). High frequency plant regeneration from leaf derived callus of high Δ9-tetrahydrocannabinol yielding Cannabis sativa L. Planta 29. Med. 76, 1629-1633. doi:10.1055/s-0030-1249773.
Lata, H., Chandra, S., Khan, I. A., and ElSohly, M. A. (2009). Thidiazuron induced high-frequency direct shoot organogenesis of Cannabis sativa L. Vitr. Cell. Dev. Biol. - Plant 45 (1), 12-19.
Lata, H., Chandra, S., Khan, I. A., and ElSohly, M. A. (2017). "Micropropagation of Cannabis sativa L.-An update," in Cannabis sativa L. - Botany and Biotechnology (Springer International Publishing), 285-297. doi:10.1007/978-3-319-54564-6_13.
Litardiere, R. D. (1925). Sur l'existence de figures didiploides dans le meristeme radiculaire du Cannabis sativa L. Cellule 35, 21-25.
Lusarkiewicz-Jarzina, A. S., Ponitka, A., and Kaczmarek, Z. (2005). Influence of cultivar, explant source and plant growth regulator on callus induction and plant regeneration of Cannabis sativa L.
Mandolino, G., and Ranalli, P. (1999). "Advances in Biotechnological Approaches for Hemp Breeding and Industry," in Advances in Hemp Research (CRC Press), 185-212. doi:10.1201/9781498705820.
Mansouri, H., and Bagheri, M. (2017). "Induction of Polyploidy and Its Effect on Cannabis sativa L.," in Cannabis sativa L. - Botany and Biotechnology. doi:10.1007/978-3-319-54564-6_17.
Miller, R. H. (1959). Morphology of Humulus lupulus. II. Secondary growth in the root and seedling vascularization. Am. J. Bot. 46, 269-277. doi:10.1002/j.1537-2197.1959.tb07012.x.
Mishchenko, S., Mokher, J., Laiko, I., Burbulis, N., Kyrychenko, H., and Dudukova, S. (2017). Phenological growth stages of hemp (Cannabis sativa L.): codification and description according to the BBCH scale. Žemés ukio Moksl. 24. doi:10.6001/zemesukiomokslai.v24i2.3496.
Movahedi, M., Ghasemi-Omran, V. O., and Torabi, S. (2015). The effect of different concentrations of TDZ and BA on in vitro regeneration of Iranian cannabis (Cannabis sativa L.) using cotyledon and epicotyl explants. 3, 20-27.
Movahedi, M., Ghasemi-Omran, V. O., and Torabi, S. (2016a). In vitro callus induction and regeneration of medicinal plant Cannabis sativa L. 32, 758-769.
Movahedi, M., Ghasemiomran, V., and Torabi, S. (2016b). Effect of explants type and plant growth regulators on in vitro callus induction and shoot regeneration of Cannabis sativa L. Iran. J. 31 Med. Aromat. Plants 32, 83-96.
Murashige, T., and Skoog, F. (1962). A revised medium for rapid growth and bio assays with tobacco tissue cultures. Physiol Plant. 15, 473- 479.
Parsons, J. L., Martin, S. L., James, T., Golenia, G., Boudko, E. A., and Hepworth, S. R. (2019). Polyploidization for the genetic improvement of Cannabis sativa. Front. Plant Sci. 10, frontio. doi:10.3389/fpls.2019.00476.
Plawuszewski, M., Lassocinski, W., and Wielgus, K. (2006). "Regeneration of Polish cultivars of monoecious hemp (Cannabis sativa L.) grown in in vitro cultures," in Renewable resources and plant biotechnology, 149-154.
Ramsay, G., and Kumar, A. (1990). Transformation of Vicia faba cotyledon and stem tissues by Agrobacterium rhizogenes: Infectivity and cytological studies. J. Exp. Bot. 41, 841-847. doi:10.1093/jxb/41.7.841.
Richez-Dumanois, C., Braut-Boucher, F., Cosson, L., and Paris, M. (1986). Multiplication vegetative in vitro du chanvre (Cannabis sativa L.). Application à la conservation des clones sélectionnés. Agron. EDP Sci. 6 (5), 487-495.
Skoog, F., and Miller, C. O. (1957). Chemical regulation of growth and organ formation in plant tissues cultured in vitro. Symp. Soc. Exp. Biol. 11, 118-30.
Tanimoto, S., and Harada, H. (1984). Roles of Auxin and Cytokinin in Organogenesis in Torenia Stem Segments Cultured in vitro. J. Plant Physiol. 115, 11-18. doi:10.1016/S0176-1617(84)80046-2.
Urits, I., Borchart, M., Hasegawa, M., Kochanski, J., Orhurhu, V., and Viswanath, O. (2019). An Update of Current Cannabis-Based Pharmaceuticals in Pain Medicine. Pain Ther. 8, 41-51. 33 doi:10.1007/s40122-019-0114-4.
Van Hieu, P. (2019). Polyploid Gene Expression and Regulation in Polysomic Polyploids. Am. J. Plant Sci. 10, 1409-1443. doi:10.4236/ajps.2019.108101.
Wahby, I., Caba, J. M., and Ligero, F. (2017). "Hairy root culture as a biotechnological tool in C. sativa," in Cannabis sativa L. - Botany and Biotechnology (Springer International Publishing), 299-317. doi:10.1007/978-3-319-54564-6_14.
Wielgus, K., Luwanska, A., Lassocinski, W., and Kaczmarek, Z. (2008). Estimation of Cannabis sativa L. tissue culture conditions essential for callus induction and plant regeneration. J. Nat. 758 Fibers 5, 199-207. doi:10.1080/15440470801976045.

## Claims

1. A method for *in vitro* direct regeneration of polyploid plants coming from *Cannabis sativa* L. by means of *in vitro* tissue culture of apical meristems, stem nodes, internodes or true leaves from a mature *Cannabis* plant or cotyledons, hypocotyls, true leaves or epicotyls from a *Cannabis* seedling, said method **characterized by**:
- comprising *in vitro* culturing of apical meristem, stem node, internode, true leaf, cotyledon, hypocotyl and/or epicotyl explants of a donor *Cannabis* plant under conditions for promoting the growing of embryos, shoots and/or shoots with roots; and
- preventing the formation of an intermediate callus phase.

2. A method for *in vitro* direct regeneration of polyploid *Cannabis* plants according to claim 1, **characterized in that** the apical meristem, stem node, internode, true leaf, cotyledon, hypocotyl or epicotyl explants are dissected from a *Cannabis* donor plant with a phenological growth stage coded from 00 to 99 according to BBCH-scale of Mishchenko *et a*/*.*, (2017).

3. A method according to any of the preceding claims, **characterized in that** the cotyledon, hypocotyl and/or epicotyl explants are dissected from a seven-days old seedlings of a *Cannabis* donor plant.

4. A method according to any of the preceding claims, **characterized in that** the culture medium for *in vitro* culturing of hypocotyl and/or epicotyl explants consists of macronutrients, micronutrients, vitamins, carbon sources and gelling agents and that is hormone-free.

5. A method according to any preceding claims, **characterized in that** the culture medium consists of macronutrients, micronutrients, vitamins and carbon sources, with or without gelling agents, plant growth regulators, chemical mutagens and chemical microtubule disruptors.

6. A method according to any preceding claim **characterized in that** the explants are grown at 22°C ± 5°C and 60% ± 10% relative humidity.

7. A method according to any preceding claim **characterized in that** the photoperiod during culturing consists of between 16 and 18 hours of light and between 6 and 8 hours of dark.

8. A method according to any preceding claim, **characterized in that** the explants are *in vitro* cultured during at least 2 weeks.

9. A method according to any preceding claim, **characterized in that** it further comprises a step prior to *in vitro* culturing of apical meristem, stem node, internode, cotyledon, true leaf, hypocotyl and/or epicotyl explants, where seeds of the donor *Cannabis* plant are surface sterilized and washed in autoclaved water.

10. A method according to claim 9, **characterized in that** once sterilized, the seeds are germinated to produce plants with a phenological growth stage coded from 00 to 99 according to BBCH-scale of Mishchenko *et a*/*.*, (2017) or seven-days-old seedlings.

11. A method according to any of claims 1 to 10 **characterized in that** it further comprises a step after culturing the explants where shoots and/or embryos regenerated from the original explant are subcultured individually to glass-tubes, vessels and/or containers of multiple volumes to produce rooted *Cannabis* plants.

12. A method according to any preceding claim **characterized by** comprising directly transplanting rooted plants obtained after *in vitro* culture of the explants in pots with fertilized substrate and subsequent acclimatization.

13. A method according to any preceding claim **characterized by** comprising a further step where *in vitro* rooted *Cannabis* plants are transplanted in pots with fertilized substrate and are subjected to acclimatization.

14. A method according to any preceding claim **characterized in that** the donor *Cannabis* plant belongs to *Cannabis sativa* L.

15. A *Cannabis* plant regenerant obtainable according to the method of any of claims 1 to 14.

16. A method for micropropagation of selected elite clones belonging to *Cannabis sativa* L. which comprises *in vitro* direct regeneration of *Cannabis* plants according to claims 1 to 14.

17. A method for obtaining polyploid *Cannabis* plants or *in vitro* regenerated *Cannabis* plants with an increased ploidy level, which comprises *in vitro* direct regeneration of a *Cannabis* plant according to claims 1 to 14.

18. A method for obtaining transgenic or gene-edited *Cannabis* plants which comprises direct *in vitro* regeneration of a *Cannabis* plant according to claims 1 to 14.

19. A method for inducing mutagenesis in order to generate variability in *Cannabis sativa* L. and produce new *Cannabis* plant genotypes which comprises direct *in vitro* regeneration of a *Cannabis* plant according to claims 1 to 14.
